Europäisches Patentamt

European Patent Office

Office européen des brevets

(11) Publication number: **0 156 633**
**B1**

(12) **EUROPEAN PATENT SPECIFICATION**

(45) Date of publication of patent specification: **28.09.88**

(21) Application number: **85302009.7**

(22) Date of filing: **22.03.85**

(51) Int. Cl.⁴: **A 61 K 39/02**

(54) Composition for prevention and treatment of mycoplasmal pneumonia.

<table>
<tr><td>(30) Priority: <b>22.03.84 JP 53497/84</b></td><td>(73) Proprietor: <b>Eisai Co., Ltd.<br>6-10, Koishikawa 4-chome Bunkyo-ku<br>Tokyo 112 (JP)</b></td></tr>
<tr><td>(43) Date of publication of application:<br><b>02.10.85 Bulletin 85/40</b></td><td rowspan="2">(72) Inventor: <b>Yamamoto, Masaichi<br>6-4-1, Saginomiya Nakano-ku<br>Tokyo (JP)</b><br>Inventor: <b>Yamamoto, Hiroshi<br>9, Mastugaoka 5-chome<br>Kagamigahara-shi Gifu Prefecture (JP)</b></td></tr>
<tr><td>(45) Publication of the grant of the patent:<br><b>28.09.88 Bulletin 88/39</b></td></tr>
<tr><td>(84) Designated Contracting States:<br><b>AT BE CH DE FR GB IT LI NL SE</b></td><td>(74) Representative: <b>Ruffles, Graham Keith et al<br>MARKS & CLERK 57-60 Lincoln's Inn Fields<br>London WC2A 3LS (GB)</b></td></tr>
<tr><td>(56) References cited:<br><b>FR-A-2 201 878</b></td><td></td></tr>
</table>

EP 0 156 633 B1

Courier Press, Leamington Spa, England.

## Description

The present invention relates to novel compositions for prevention and treatment of mycoplasmal pneumonia.

Mycoplasmal pneumonia is contracted generally by humans and animals and is induced by pathogenic bacteria of the genus *Mycoplasma*. A characteristic feature of mycoplasmal pneumonia is that the particular *Mycoplasma* as pathogenic bacterium varies depending upon the species of animal. Accordingly, in the prevention and treatment of mycoplasmal pneumonia, complicated and appropriate procedures have to be adopted which depend upon the animal susceptible to *Mycoplasma*. Such is the case particularly in the livestock industry.

*Mycoplasma* bacteria have been isolated and studied, and the distribution and ecology has been clarified only recently. However, nothing has yet been satisfactorily established in the way of appropriate treatment for prevention and therapy. Prevention has been attempted by vaccination or by chemotherapy using antibiotics such as tetracycline, tylosin, and other drugs, but according to reports, beneficial effects have not been obvious from these treatments. Particularly in recent years, much has been reported on attempts at immunological therapy and prevention by administration of a vaccine of killed *Mycoplasma*. However, satisfactory results have not yet been observed in practical situations. Immunity through vaccination with killed *Mycoplasma* is described in the following publications, listed below for reference.

1) Immunogenic Potency of Oil-Emulsified *M. gallisepticum* Bacterin; *Avian Dis.,* vol. 25, 821—826 (1981).
2) Some Experiments Relating to Artificial Immunity in Enzootic Pneumonia of Pigs; *J. Hyg., Camb.,* vol. 67 456—476 (1969).
3) Mycoplasmal Pneumonia of Swine: Active and Passive Immunizations; *Am. J. Vet. Res.,* vol. 31, 1737—1741 (1971).
4) Immunization Against *Mycoplasma* Infections of Poultry; *Am. J. Vet. Res.,* vol. 21, 482—485 (1960).
5) Local Immunization in Chicken Respiratory Tract with Killed *Mycoplasma gallisepticum* Vaccine; *Jap. J. Vet. Sci.,* vol. 36, 311—319 (1974).
6) Acquired Immunity to *Mycoplasma pneumoniae* Pneumonia in Hamster; *Microbiol. Immunol.,* vol. 22, 181—195 (1978).
7) Immunoprophylaxis of Experimental *Mycoplasma pneumoniae* Disease; *Infect. Immunity,* vol. 16, 88—92 (1977).
8) Protective Effect of Vaccines in Experimental *M. pneumoniae* Disease; *Infect. Immunity,* vol. 1, 559—565 (1970).
9) Induction of Immunity in Calves to *Mycoplasma bovis* Infection of the Respiratory Tract; *Vet. Microbiol.,* vol. 2, 29—37 (1977).
10) Immunity to *Mycoplasma bovis* Infections of the Respiratory Tract of Calves; *Res. Vet. Sci.,* vol. 28, 242—249 (1979).
11) Enzootic Pneumonia of Pigs: Immunization Attempts Inoculating *M. suipnumoniae* Antigen by Various Routes and with Different Adjuvants; *Brit. Vet. J.,* Vol. 129, 456—464 (1975).
12) Cell-mediated and Humoral Immune Response in Swine after Vaccination and Natural Infection with *Mycoplasma hyopneumoniae; Am. J. Vet. Res.,* vol. 42, 784—788 (1981).

European Patent Application No. 28814 describes the use of abrin as effective immunostimulant in the treatment of cancer and viral bacterial infections. Similarly, European Patent Application No. 28815 ricin as an immunostimulant.

In view of the existing situation, there is a need for novel compositions for prevention and treatment of mycoplasmal pneumonia.

The present invention provides compositions for prevention and treatment of mycoplasmal pneumonia. The compositions of this invention comprise killed *Mycoplasma* vaccine together with ricin and/or abrin. The ricin may be whole ricin or a crosslinked ricin such as crosslinked ricin D.

*Mycoplasma* bacteria are exemplified by *Mycoplasma mycoides* var. *mycoides, Mycoplasma hyopneumoniae, Mycoplasma pneumoniae, Mycoplasma gallisepticum, Mycoplasma dispar, Mycoplasma pulmonis, Mycoplasma mycoides* var. *capri* and others.

The classification, nomenclature, general properties and distribution of *Mycoplasma* have finally been clarified recently, and definitive description has now been given. Reviews on *Mycoplasma* appear in the publications listed below. Thus, the reader is referred to this literature for descriptions explaining the classification, nomenclature, general properties and distribution of *Mycoplasma*.

13) Shmuel Razin: The *Mycoplasma* Microbiological Reviews, June 1978, vol. 42, No. 2, p. 414—470.
14) "New Taxology of Microorganisms" edited by Takeharu Hasegawa, published by Kodan-sha, pages 31—50, *Mycoplasma*.

The killed *Mycoplasma* vaccine for a composition in accordance with the present invention is suitably a product obtained by subjecting *Mycoplasma* bacteria to conventional vaccine preparation. Such killed *Mycoplasma* vaccine can be obtained, for instance, by adding sodium azide to *Mycoplasma* bacteria, incubating the resulting mixture and then storing it at about 4°C to freeze the bacteria.

The ricin or whole ricin used in the present invention comprises a lectin isolated from the seeds of castor oil plant, which plant belongs to the family Euphorbiaceae. The lectin is a sugar protein having a

2

molecular weight of about 64,000 to 65,000. It consists structurally of an A chain having a molecular weight of the order of about 31,000 and a B chain having a molecular weight of the order of about 33,000, the A and B chains being connected by a single —S—S— bond. It is already known in Japanese Patent Application Laid-open (kokai) No. 68,615/81 that ricin possesses an immunopotentiation effect. However, it has been newly discovered for the present invention that by administering ricin (the whole ricin or crosslinked ricin) or abrin, along with killed *Mycoplasma* vaccine, the ricin or abrin exhibits an advantage effect in preventing and treating mycoplasmal pneumonia.

The abrin for use in this invention comprises a lectin isolated from the seeds of jequirity (*Abrus precatorius*) which is a plant of the family Leguminoseae occurring naturally in the tropical and subtropical zones. The lectin is a kind of glycoprotein having a molecular weight of about 65,000.

Both ricin and abrin are stable. In particular, when they are stored at a temperature of about 4°C in an aqueous solution of around pH 6.0, no change in activity is observed for several years. They can also be subjected to lyophilization.

Crosslinked ricin D for use in this invention can be prepared for instance in accordance with the disclosure in Agric. Biol. Chem., 43 (3), 547—554, 1979.

In the present invention, it is appropriate for the dosage of killed *Mycoplasma* vaccine to be generally in the range of $10^5$ to $10^{10}$ (bacterial count) for each human or other animal.

The minimum dosage of ricin or abrin is generally about 3 ng per each human or each animal, irrespective of body weight. On the other hand, the maximum dosage is generally about 1 to 1.5 µg/kg. Considering that the acute toxicity ($LD_{50}$) of ricin or abrin is around 12 µg/kg (mouse, i.p.), the aforesaid dosage range is extremely low as compared to the acute toxicity.

As has been mentioned above, in mycoplasmal pneumonia to which the present invention applies, the kind of *Mycoplasma* bacteria differs depending upon the species of animal which has caught the disease. Accordingly, the development, clinical findings, clinical pathology autopsy findings, diagnosis, etc., are different for each species. In the following publications, mention is made of different mycoplasmal pneumonia which differ depending upon the species of animal. The reader is referred to this literature.

15) Rinsho Jugaku (Clinical Veterinary Science), published by Bun-Ei-Do, pages 625—635.
16) Tonbyo-Gaku (Swine Disease), published by Kindai Shuppan Co., Ltd., 1982, pages 535—548.
17) Jui Densenbyo-gaku (Veterinary Infectious Disease), published by Kindai Shuppan Co., Ltd., 1979, pages 376—378 and 227—229.
18) Gyubyo-gaku (Cow Disease), published by Kindai Shuppan Co., Ltd., 1980, pages 582—587.
19) Kansensho-gaku (Infectious Disease), *Kiso-to-Rinsho*, published by Medical Review Co., Ltd., 1981, pages 635—639.
20) Keibyo Zusetsu (Illustrated Chicken Disease), published by Nippon Chikusan Shinko Kai, pages 187—211.

The compositions of the present invention are generally administered subcutaneously, intramuscularly, intraperitoneally, intravenously or intrathoracically. Furthermore, depending upon the case, it is also possible to administer percutaneously.

Referring to an illustrative embodiment of administration in the form of an injection, the composition of the present invention may be an injection in which killed *Mycoplasma* vaccine and ricin are jointly formulated. Alternatively, killed *Mycoplasma* vaccine and ricin may be prepared for independent injection and the injections may then be used in combination. For this alternative, the injections are used either after mixing them or they are administered sequentially with a suitable interval of time. To obtain injectable formulations, the ingredients can be dissolved in a buffered solution having a neutral or weakly acidic character and the solution used to charge an ampoule. Lyophilization can be employed after charging the ampoule.

The present invention will be decribed by way of illustration with reference to the following Experiments, in which reference is made to the accompanying drawings.

Figure 1 is a graph showing change of body weight ratio with the passage of time, for the Experiment 1.

Figure 2 is a graph showing the relationship between antibody titer and dosage of ricin, for the Experiment 2.

Figures 3, 4 and 5 are graphs showing the relationship between swelling rate and each sample, respectively for the Experiments 2, 3 and 4.

Experiment 1

*Mycoplasma pulmonis* 925 T strain was incubated in a liquid medium for 3 days, followed by washing 3 times with a 0.9% saline solution. Sodium azide was added to the culture solution to a level of 0.1%. The mixture was incubated at 37°C for 30 minutes and then stored at 4°C. The resultant solution was diluted to an $OD_{620\,nm}$ of 1.0, to give a killed *Mycoplasma* vaccine. To 0.1 ml of the killed *Mycoplasma* vaccine, 0.1 ml of a ricin solution of known concentration was added to prepare solutions containing 0.1 ng, 1.0 ng and 10 ng of ricin, which were respectively labelled Sample A, B and C.

ICR mice of age about 10 weeks were divided into 4 groups, to give a group receiving Sample A, a group receiving Sample B, a group receiving Sample C and a control group to which no sample was administered. The administration was by subcutaneous injection at the cervical region of the mice. Then, the head of each mouse was immersed in a liquid culture ($10^8$ ccu/ml) of *Mycoplasma pulmonis* for 5 seconds at 1 week after the injection, whereby infection with mycoplasmal pneumonia was made possible. After the infection, the mice were fed for 6 weeks, during which changes in body weight, the number of dead mice, and the number of mice with lesions were determined. A further 6 weeks later, the mice were sacrificed for autopsy, and the ratio of pulmonary lobes with lesion, the number of mice from which *Mycoplasma pulmonis* was isolated, and the isolated bacterial count were determined.

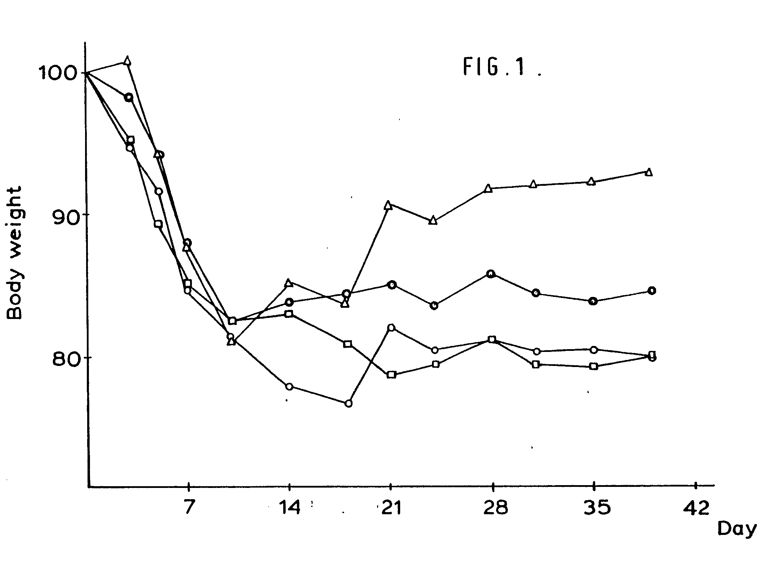

The results are shown in Figure 1, which shows change in the body weight ratio against time in days. The points marked with light open circles correspond with the results when administering Sample A, the points marked with heavy open circles correspond with the results when administering Sample B, the points marked with open triangles correspond with the results when administering Sample C, and the points marked with open squares correspond with the results for the control group.

From Figure 1, it is to be noted that for the control group and the group receiving Sample A, the mice were infected and grew weak to cause a marked reduction in body weight. In the group receiving Sample B and the group receiving Sample C, the reduction in body weight caused by growing weak was later restored.

Results for this Experiment are also shown in the following Table 1, which includes the number of dead mice and the number of mice with lesions at 6 weeks and also gives various infection indices at the 6th week.

TABLE 1

| Group | Number of mice | Number of dead mice | Number of mice with lesions | Ratio of pulmonary lobe with lesions | Isolation of *M. pulmonis* Number of mice | CCU count |
|---|---|---|---|---|---|---|
| Sample A | 10 | 3 | 4 (40.0%) | 20/50 (40.0%) | 9 (90.0%) | $10^{4.6}$ |
| Sample B | 10 | 0 | 0 (0%) | 0/50 (0%) | 8 (80.0%) | $10^{1.6}$ |
| Sample C | 8 | 1 | 2 (25.0%) | 3/40 (7.5%) | 4 (50.0%) | $10^{2.1}$ |
| Control | 10 | 3 | 7 (70.0%) | 29/50 (58.0%) | 10 (100.0%) | $10^{5.9}$ |

From Table 1, it can be seen that the composition of the present invention shows an effect of preventing infection by *Mycoplasma pulmonis*.

Experiment 2

Samples A, B and C used in Experiment 1 were likewise used as samples. For control samples, Sample D and Sample E were prepared. Sample D was a sample containing killed vaccine alone, which was prepared by adding 0.1 ml of water to 0.1 ml of the killed vaccine solution of Experiment 1. Sample E was a sample containing ricin alone, which was prepared by adding 0.1 ml of ricin solution to 0.1 ml of water to give a solution containing 1.0 ng of ricin.

ICR mice were divided into 5 groups, each of 5 mice. Each of the samples were subcutaneously administered to the mice by injection at the cervical region. Blood was collected 2 weeks after the administration of the sample. The serum was diluted and the antibody titer was determined by an agglutination reaction. Furthermore, a DTH test was performed 1 week after the administration of the samples in order to determine a swelling ratio. That is, physiological saline was administered to the left limb pad and the killed vaccine of Example 1 was given to the right limb pad. Swelling after 24 hours was measured and, a swelling rate was determined by the following equation.

$$\text{Swelling rate } (\%) = \frac{\text{Swell (R)} - \text{swell (L)}}{\text{Swell (L)}} \times 100$$

where "swell (R)" is the swelling at the right limb pad, and "swell (L)" is the swelling at the left limb pad.

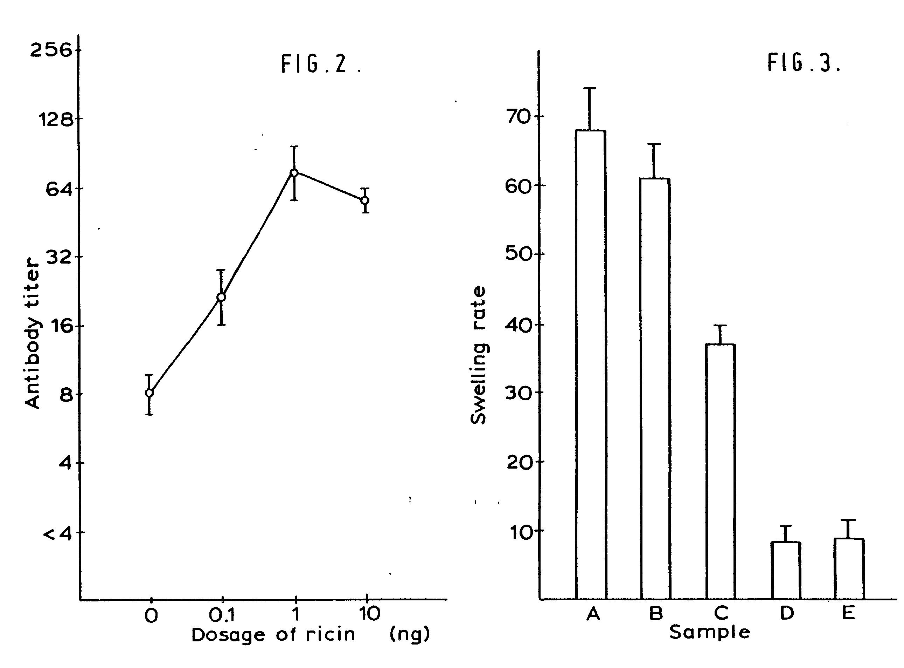

The results are shown in Figures 2 and 3.

Figure 2 shows the relationship between antibody titer and dosage of ricin, where the points marked with light open circles correspond with the results when administering Samples A, B, C and D, and the points marked with solid circles correspond with the results when administering Sample E. From Figure 2, it is noted that by adding ricin to *Mycoplasma pulmonis* killed vaccine, a high antibody titer is obtained.

Figure 3 shows the relationship between swelling rate for the respective samples. The difference in swelling rates between each of Samples A and D, Samples B and D and Samples C and D is significant at $p < 0.01$. From Figure 3, it is noted that by adding ricin to *Mycoplasma pulmonis* killed vaccine, the DTH reaction increases depending on the dosage of ricin, and humoral and cellular immune response are accelerated.

4

Experiment 3

Experiments 1 and 2 were repeated, except that the ricin was replaced by abrin. Samples A, B and C were thus prepared as the solutions containing 0.1 ng, 1.0 ng and 10 ng of abrin, respectively. A control sample, Sample D containing killed vaccine alone, was also prepared.

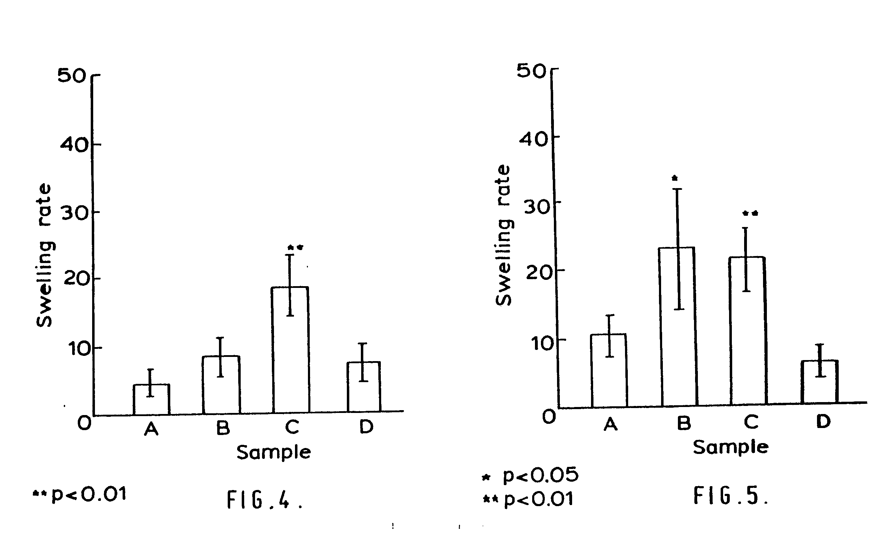

Figure 4 shows the relationship between swelling rate for the respective samples.

The difference of swelling rates between Samples C and D is significant at $p < 0.01$. From Figure 4, it is noted that by the addition of abrin to *Mycoplasma pulmonis* killed vaccine, the DTH reaction increases depending on the dosage of abrin, and cellular immune response is accelerated.

Experiment 4

Experiments 1 and 2 were repeated, except that crosslinked ricin D was used in place of the ricin. Samples A, B and C were thus prepared as the solutions containing 0.1 µg, 1.0 µg and 10 µg of crosslinked ricin D, respectively. A control sample, Sample D containing killed vaccine alone, was also prepared.

Figure 5 shows the relationship between swelling rate for the respective samples.

The difference in swelling rates bewteen Samples B and D is significant at $p < 0.05$, and that between Samples C and D is significant at $p < 0.01$. From Figure 5, it is noted that by virtue of the addition of crosslinked ricin D to *Mycoplasma pulmonis* killed vaccine, the DTH reaction increases depending on the dosage of crosslinked ricin D, and the cellular immune response is accelerated.

The present invention will be described more concretely with reference to the following non-limiting Examples.

Example 1

*Mycoplasma pulmonis* 925T strain was incubated in liquid medium for 3 days and then washed with a 0.9% aqueous saline solution 3 times. Sodium azide was added to a concentration of 0.01%, and the resulting mixture was incubated at 37°C for 30 minutes. The thus obtained solution was diluted to an $OD_{620\,nm}$ of 1.0. The diluted solution was charged in to an ampoule, giving a killed vaccine ampoule.

Separately, a ricin-containing ampoule was prepared. Namely, 0.05 ml of glacial acetic acid was added to 5 ml of a suspension containing ricin crystals at 10 mg/ml thereby to dissolve the ricin crystals. To the thus obtained solution, 10 ml of 0.01M phosphate buffered solution (pH 6.0, hereafter referred to as PBS (pH 6.0)) containing 0.15M saline was added and 0.4 ml of 1M $Na_2HPO_4$ was further added to adjust the pH to about 5. The mixture was centrifuged at 15,000 rpm for 10 minutes. The supernatant was recovered and filtered. The filtrate was put in a cellophane (trade mark) tube, which was subjected to dialysis overnight using 5 liters of PBS (pH 6.0) as the outer dialysis solution. Setting the 1% $E_{620\,nm}$ for the ricin specimen at 11.8 (Olsnes *et al., J. Biol. Chem.,* 249, 803—810 (1974)), the internal dialysis solution was diluted with PBS (pH 6.0) to give a ricin solution of 500 µg/ml. A glass ampoule of 1 ml volume was charged with 0.1 ml of the solution and lyophilized to prepare an ampoule containing 50 µg of ricin. A pair of the aforesaid killed vaccine ampoule and the ricin-containing ampoule was obtained as a composition for prevention and treatment in accordance with the present invention.

Example 2

In the same manner as in Example 1, an abrin-containing ampoule was prepared to give a composition for prevention and treatment of the present invention, by the use of abrin in place of ricin.

Example 3

Example 1 was repeated, except that crosslinked ricin D was employed instead of ricin. There was thus obtained a crosslinked ricin D-containing ampoule for use as part of a composition of the present invention for prevention and treatment.

**Claims**

1. A composition for prevention and treatment of mycoplasmal pneumonia, the composition comprising killed *Mycoplasma* vaccine together with ricin or abrin.

2. A composition according to claim 1, wherein the ricin is whole ricin or crosslinked ricin.

3. A composition according to claim 1 or 2, wherein the unit dose of the killed *Mycoplasma* vaccine is in the range of $10^5$ to $10^{10}$ (bacterial count).

4. A composition according to claim 1, 2 or 3, wherein the minimum unit dose of ricin or abrin is 3 ng.

5. A composition according to any of claims 1 to 4, wherein the maximum unit dose of ricin or abrin is 1 or 1.5 µg/kg.

6. A composition according to any of claims 1 to 5, wherein the killed *Mycoplasma* vaccine and the ricin or abrin are formulated for simultaneous or sequential administration.

7. A composition according to any of claims 1 to 6, which is in the form of an injection containing killed *Mycoplasma* vaccine and ricin or abrin.

8. A composition according to any of claims 1 to 6, which is in the form of an injection containing killed *Mycoplasma* and a separate injection containing ricin or abrin.

9. The use of killed *Mycoplasma* vaccine, ricin and/or abrin in the preparation of a medicament for prophylaxis or therapy of mycoplasmal pneumonia.

10. A method of preparing a pharmaceutical composition for use in therapeutic treatment of mycoplasmal pneumonia, in which ricin and/or abrin and killed *Mycoplasma* vaccine are formulated for separate administration.

**Patentansprüche**

1. Zusammensetzung zur Vorbeugung und Behandlung von Mycoplasma-Pneumonie, dadurch gekennzeichnet, daß sie abgetöte Mycoplasma-Vakzine zusammen mit Ricin oder Abrin enthält.

2. Zusammensetzung nach Anspruch 1, dadurch gekennzeichnet, daß das Ricin als solches oder quervernetzt vorliegt.

3. Zusammensetzung nach Anspruch 1 oder 2, dadurch gekennzeichnet, daß die Einheitsdosis an abgetöteter Mycoplasma-Vakzine im Bereich von $10^5$—$10^{10}$ (Bakterienzahl) liegt.

4. Zusammensetzung nach Anspruch 1, 2 oder 3, dadurch gekennzeichnet, daß die Minimaldosis von Ricin oder Abrin 3 ng beträgt.

5. Zusammensetzung nach einem der Ansprüche 1 bis 4, dadurch gekennzeichnet, daß die Maximaldosis von Ricin oder Abrin 1 bzw. 1,5 µg/kg beträgt.

6. Zusammensetzung nach einem der Ansprüche 1 bis 5, dadurch gekennzeichnet, daß die abgetötete Mycoplasma-Vakzine und das Ricin oder Abrin zur gleichzeitigen oder aufeinanderfolgenden Verabreichung formuliert sind.

7. Zusammensetzung nach einem der Ansprüche 1 bis 6, dadurch gekennzeichnet, daß sie als injizierbare Formulierung, die abgetötete Mycoplasma-Vakzine und Ricin oder Abrin enthält, vorliegt.

8. Zusammensetzung nach einem der Ansprüche 1 bis 6, dadurch gekennzeichnet, daß sie als injizierbare Formulierung, die abgetötetes Mycoplasma enthält, und als eine getrennte injizierbare Formulierung, die Ricin oder Abrin enthält, vorliegt.

9. Verwendung von abgetöteter Mycoplasma-Vakzine, Ricin und/oder Abrin zur Herstellung eines Arzneimittels zur Vorbeugung oder Behandlung von Mycoplasma-Pneumonie.

10. Verfahren zur Herstellung einer pharmazeutischen Zusammensetzung zur therapeutischen Behandlung von Mycoplasma-Pneumonie, dadurch gekennzeichnet, daß Ricin und/oder Abrin und abgetötete Mycoplasma-Vakzine zur getrennten Verabreichung formuliert werden.

**Revendications**

1. Une composition pour la prévention et le traitement de la pneumonie à mycoplasmes, cette composition comprenant un vaccin fait de *Mycoplasma* tués avec de la ricine ou de l'abrine.

2. Une composition selon la revendication 1, dans laquelle la ricine est de la ricine entière ou de la ricine réticulée.

3. Une composition selon la revendication 1 ou 2, dans laquelle la dose unitaire de vaccin à base de *Mycoplasma* tués est dans la gamme de $10^5$ à $10^{10}$ (numération bactérienne).

4. Une composition selon la revendication 1, 2 ou 3, dans laquelle la dose unitaire minimale de ricine ou d'abrine est de 3 ng.

5. Une composition selon l'une quelconque des revendications 1 à 4, dans laquelle la dose unitaire maximale de ricine ou d'abrine est de 1 ou 1,5 µg/kg.

6. Une composition selon l'une quelconque des revendications 1 à 5, dans laquelle le vaccin fait de *Mycoplasma* tués et la ricine ou l'abrine sont présentés pour l'administration simultanée ou successive.

7. Une composition selon l'une quelconque des revendications 1 à 6, qui est sous forme d'une injection contenant un vaccin fait de *Mycoplasma* tués et de la ricine ou de l'abrine.

8. Une composition selon l'une quelconque des revendications 1 à 6, qui est sous forme d'une injection contenant des *Mycoplasma* tués et d'une injection séparée contenant de la ricine ou de l'abrine.

9. L'utilisation de vaccin fait de *Mycoplasma* tués, de ricine et/ou d'abrine dans la préparation d'un médicament pour la prophylaxie ou la thérapeutique de la pneumonie à mycoplasmes.

10. Un procédé de préparation d'une composition pharmaceutique pour l'utilisation dans le traitement thérapeutique de la pneumonie à mycoplasmes, dans lequel on présente pour l'administration séparée de la ricine et/ou de l'abrine et un vaccin fait de *Mycoplasma* tués.

FIG.1.

FIG.2.

FIG.3.

0 156 633

FIG.5.

Swelling rate

Sample

* p<0.05
** p<0.01

FIG.4.

Swelling rate

Sample

** p<0.01